# DEMANDE DE BREVET EUROPEEN

(11) **EP 3 708 143 A1**
(43) Date de publication de la demande: **16.09.2020**
(21) Numéro de dépôt: 20160233.1
(22) Date de dépôt: 27.12.2012
(51) Int. Cl.: A61K 8/06, A61Q 9/00, A61Q 19/00, A61K 8/60, A61K 8/92

(54) **PROCÉDÉ DE FABRICATION D'UNE ÉMULSION AQUEUSE D'UNE SUBSTANCE ACTIVE HUILEUSE POUR APPLICATION COSMÉTIQUE, ALIMENTAIRE OU PHARMACEUTIQUE**

(62) Demande divisionnaire de: 12813375.8
(71) Demandeur: Epiphani, Jean-Claude, 20166 Porticcio (FR); AR2I, 92350 Le Plessis-Robinson (FR)
(72) Inventeur: EPIPHANI, Jean-Claude, 20166 PORTICCIO (FR)
(74) Mandataire: Gevers & Orès

(57) **Abrégé**

Le procédé comprend les étapes suivantes :
- mélange de composants hydrophiles, comprenant de l'eau, un saponoside émulsionnant, celui-ci représentant moins de 1 % de l'émulsion quel que soit la proportion de phase huileuse
- mélange de composants lipophiles, comprenant la substance active huileuse, celle-ci pouvant atteindre 40 % de l'émulsion,
- homogénéisation n° 1 des deux phases précédentes à l'aide d'un mélangeur du type rotor-stator, ou à ultra-sons, jusqu'à l'obtention d'un diamètre moyen des gouttelettes huileuses inférieur à 6 µm
- homogénéisation n° 2 du mélange précédent à l'aide d'un homogénéiseur à haute pression, jusqu'à l'obtention d'un diamètre moyen des gouttelettes huileuses inférieur à 0,4 µm.

## Description

### 1. Champ de l'invention

La présente invention est relative à des émulsions aqueuses de substances actives huileuses destinées aux industries cosmétiques, alimentaires et pharmaceutiques.

### 2. Etat actuel de la technique

Dans les domaines des industries cosmétiques, alimentaires et pharmaceutiques, de nombreuses substances actives sont de type huileux, c'est à dire lipophiles ou hydrophobes : huiles végétales, huiles essentielles, concentrés de parfums, actifs huileux divers de type cosmétique, alimentaire ou pharmaceutique. Il est très intéressant de réaliser des émulsions aqueuses de ces substances actives de type huileux, pour une raison essentielle : l'eau est le vecteur le mieux adapté pour le corps humain, que ce soit pour un usage externe (parfums et applications cosmétiques) ou pour un usage interne (applications alimentaires ou pharmaceutiques).On sait depuis longtemps réaliser des émulsions « huile dans eau » à l'aide d'émulsionnants chimiques divers, mais ces émulsionnants présentent des inconvénients notoires : ils sont généralement mal supportés par le corps humain,- ils doivent être utilisés avec une concentration importante par rapport aux substances actives. La technologie des cyclodextrines est une autre voie possible, mais elle présente aussi plusieurs contraintes qui limitent fortement son utilisation.On a d'autre part envisagé l'utilisation des saponosides, qui sont des émulsionnants végétaux appartenant à la famille des glucides (hétérosides).Une première catégorie d'applications utilise des substances auxiliaires (hydrates de carbone, éthanol,...) en quantités importantes qui dénaturent l'émulsion finale.Un deuxième mode d'applications n'utilise pas de substances auxiliaires, ainsi que le dispose le brevet FR 02 14436, mais il ne permet pas en pratique de dépasser sensiblement, avec une fluidité et une stabilité satisfaisantes, une concentration en phase huileuse de 30 % de l'émulsion, ce qui est une limitation gênante, en particulier pour certaines applications cosmétiques.

Aucun procédé connu à ce jour ne permet de réaliser des émulsions aqueuses fluides et stables contenant plus de 40 % de phase huileuse, avec moins de 1 % d'émulsionnant végétal.

### 3. Description de l'invention

Pour pallier les inconvénients précédents le demandeur, partant du procédé décrit par le brevet FR 02 14436, a exploré plusieurs voies, et en particulier :
- l'utilisation d'additifs (coémulsionnants, ...)
- l'emploi de tous les moyens connus de mélange par action mécanique
- la mise en œuvre de modes opératoires divers.

Aucune voie utilisée seule ne permet d'atteindre le but recherché.

Après plusieurs années de recherches et d'essais, il a été mis au point par le demandeur un procédé permettant de réaliser des émulsions aqueuses de substances actives huileuses, dans les domaines des industries cosmétiques, alimentaires et pharmaceutiques, le dit procédé éliminant les inconvénients des techniques en vigueur jusqu'à maintenant et permettant de réaliser des émulsions aqueuses fluides et stables contenant plus de 40 % de phase huileuse, avec moins de 1 % d'émulsionnant végétal.Ainsi l'invention concerne un procédé de fabrication d'une émulsion aqueuse d'une substance active huileuse pour application cosmétique, alimentaire ou pharmaceutique, comprenant les étapes suivantes :
- mélange de composants hydrophiles, comprenant de l'eau, un saponoside émulsionnant, celui-ci représentant moins de 1 % de l'émulsion quel que soit la proportion de phase huileuse
- mélange de composants lipophiles, comprenant la substance active huileuse, celle-ci pouvant atteindre 40 % de l'émulsion,
- homogénéisation n° 1 des deux phases précédentes à l'aide d'un mélangeur du type rotor-stator, ou à ultra-sons, jusqu'à l'obtention d'un diamètre moyen des gouttelettes huileuses inférieur à 6 µm
- homogénéisation n° 2 du mélange précédent à l'aide d'un homogénéiseur à haute pression, jusqu'à l'obtention d'un diamètre moyen des gouttelettes huileuses inférieur à 0,4 µm.

La détermination du diamètre moyen des gouttelettes huileuses est effectuée par une mesure classique de granulométrie.

La concentration optimale en émulsionnant et les paramètres de fonctionnement des homogénéiseurs doivent être déterminés par des essais préalables, vu la diversité des substances actives huileuses à émulsionner, des concentrations requises et des appareils utilisables.

Seule la combinaison « saponosides + homogénéisation n° 1 + homogénéisation n° 2 » permet d'obtenir une émulsion aqueuse fluide et stable pouvant comprendre jusqu'à 40 % de phase huileuse, avec moins de 1 % d'émulsionnant végétal.
Ainsi l'utilisation d'un autre émulsionnant, ou l'absence d'une des 2 étapes d'homogénéisation, ou le non-respect des valeurs indiquées, ne permettent pas d'atteindre le résultat mentionné.
Il n'était pas du tout évident que la combinaison de ces 3 éléments permette d'atteindre le but recherché. D'ailleurs cela n'avait jamais été mis en œuvre auparavant.
Voici quelques résultats observés avec des combinaisons différentes de celles de l'invention, en prenant comme base l'exemple mentionné au paragraphe 4 ci-dessous.
- Si on utilise un émulsionnant chimique performant, par exemple à base d'esters polyglycériques, il n'est pas possible d'utiliser moins de 3 % d'émulsionnant, toutes choses égales par ailleurs.
- Si on n'effectue pas l'une des deux homogénéisations, ou qu'on ne respecte pas les limites indiquées pour la finesse de l'émulsion, on ne réussit pas à atteindre la valeur de 40 % de phase huileuse avec 1 % de saponosides : une partie plus ou moins importante de la phase huileuse apparaît rapidement à la surface de l'émulsion, ce qui rend celle-ci impropre à l'utilisation.

Avantageusement, on ajoute au mélange de composants hydrophiles au moins un additif hydrophile, et au mélange de composants lipophiles, au moins un additif lipophile.

On entend par « additifs », des substances annexes telles que des conservateurs, des anti-oxydants, des séquestrants, etc ..., assurant la conservation de la substance active.

En lieu et place des additifs, on peut utiliser tout autre moyen de conservation, comme par exemple une étape de stérilisation de l'ensemble de l'émulsion homogénéisée.

Les émulsions obtenues sont toujours laiteuses.
Dans certaines applications, il est possible ou souhaitable d'utiliser en sus des saponosides au moins un coémulsionnant d'origine naturelle ou synthétique.

Il faut noter enfin que les émulsions de l'invention, très fluides, peuvent servir de base pour la réalisation de gels ou crèmes par addition d'épaississant(s) adaptés à l'application.

### 4. Exemple de fabrication d'une émulsion selon le procédé de l'invention

Il s'agit de réaliser une émulsion aqueuse d'huiles végétales à 40 %.
Les composants de l'émulsion, et leurs % masse, sont les suivants.
Composants hydrophiles- Eau déminéralisée (phase continue) :58,7 %- Saponosides purs (émulsionnant) :0,8 %Composants lipophiles- Huiles végétales (phase dispersée) :40,0 %- Microcare MTD2 de Thor (conservateur) :0,5 %Total :100,0 % On mélange séparément les composants hydrophiles et les composants lipophiles pour obtenir des phases homogènes.On effectue l'homogénéisation n° 1 des deux phases avec un mélangeur de la marque protégée Ultra-Turrax, du type rotor-stator, à 20.000 tours / minute. On effectue l'homogénéisation n° 2 du mélange précédent avec un homogénéiseur à haute pression de la marque protégée APV, en recyclage, avec une pression de service de 600 bars.

On obtient une émulsion laiteuse fluide et stable.
Les temps d'homogénéisation doivent être déterminés expérimentalement et dépendent de la nature des huiles végétales et des caractéristiques des homogénéiseurs.
Voici les valeurs pour deux mélanges différents, avec les appareils mentionnés ci-dessus, pour des lots de 1 kg chacun.
- Mélange a : huile de tournesol 50 % + huile d'olive 50 %
- Mélange b : huile de tournesol 25 % + oléine de Karité 35 % + huile d'argan 40 %

Temps minima d'homogénéisation

| | Homogénéisation n° 1 | Homogénéisation n° 2 |
|---|---|---|
| - Mélange a | 10 minutes | 16 minutes |
| - Mélange b | 12 minutes | 22 minutes |

## Revendications

1. Procédé de fabrication d'une émulsion aqueuse d'une substance huileuse comprenant les étapes suivantes:
a) mélange des composants hydrophiles comprenant de l'eau,
b) mélange des composants lipophiles comprenant la substance huileuse,
c) homogénéisation n°1 des deux phases obtenues à l'étape a) et à l'étape b) en présence d'un émulsionnant, à l'aide d'un mélangeur du type rotor stator, ou à ultra-sons jusqu'à l'obtention d'un diamètre moyen des gouttelettes huileuses inférieur à 6 µm,
d) homogénéisation n°2 du mélange obtenu à l'étape c) à l'aide d'un homogénéiseur à haute pression, jusqu'à l'obtention d'un diamètre moyen des gouttelettes huileuses inférieur à 0,4 µm,
l'émulsion obtenue comprenant jusqu'à 40% en masse, par rapport à sa masse totale, de substance huileuse, et
l'émulsionnant consistant en des saponosides ajouté dans les composants de l'étape a) en un pourcentage en masse, par rapport à la masse totale de l'émulsion, inférieur à 1.

2. Procédé selon la revendication 1, comprenant de plus l'ajout d'au moins un agent hydrophile dans le mélange de l'étape a) et l'ajout d'au moins un composant lipophile à l'étape b).

3. Procédé selon la revendication 1 comprenant de plus une étape e) de stérilisation de l'émulsion obtenue après l'étape d).

4. Emulsion aqueuse obtenue par le procédé selon l'une des revendications 1 à 3 comprenant :
- jusqu'à 40% en masse, par rapport à sa masse totale, de substance huileuse,
- moins de 1% en masse d'un saponoside,
- des gouttelettes huileuses ayant un diamètre moyen inférieur à 0,4 µm.

5. Gel comprenant l'émulsion selon la revendication 4.

6. Crème comprenant l'émulsion selon la revendication 4.
